# EUROPEAN PATENT APPLICATION

(11) **EP 4 574 191 A2**
(43) Date of publication of application: **25.06.2025**
(21) Application number: 25159573.2
(22) Date of filing: 25.11.2014
(51) Int. Cl.: A61M 15/06

(54) **PULMONARY DELIVERY DEVICES**

(30) Priority: 26.11.2013 GB 201320834; 04.03.2014 GB 201403827; 04.03.2014 GB 201403828; 02.04.2014 GB 201405979; 28.10.2014 GB 201419148
(62) Divisional of application: 21188785.6
(71) Applicant: Twenty Sixteen (2016) Pharma Limited, Liverpool, Merseyside L3 5TF (GB)
(72) Inventor: Pandya, Anant, Croyden, CR0 5DJ (GB); Gallagher, George, Caerwys, CH7 5PY (GB)
(74) Representative: Lees, Kate Jane

(57) **Abstract**

A pulmonary delivery apparatus (10) comprising: a first chamber (15) adapted to thermally vaporise a quantity of a first liquid to form a relatively warm first vapour (B) and a second chamber (16) adapted to atomize a quantity of a second liquid without heating of the second liquid to form a mist of a relatively cold second vapour (A), and an outlet (30) via which, in use, a user can inhale a mixture of the first and second vapours. An active ingredient such as nicotine is provided in the second chamber and an inert liquid in the first chamber.

## Description

This invention relates to pulmonary delivery devices, particularly but not exclusively, to pulmonary delivery devices suitable for delivering active molecules and/or medicaments to users, such as nicotine.

### TECHNICAL FIELD.

Pulmonary delivery devices have widespread uses in modern medicine as they enable drugs and medicaments to be delivered directly to the user's lungs. Moreover, as medicaments delivered to the lungs enter the bloodstream directly, rather than via the body's metabolism, as is the case with oral delivery systems, the benefits of the medicament, especially in pain relief or drug-weaning applications, are felt by the user almost immediately. Another major benefit of pulmonary delivery devices is their ability to deliver drugs without the use of needles.

Existing pulmonary delivery systems take various forms, including inhalator sprays, nebulisers, metered dose inhalers in which the medication is administered in the form of a mist inhaled by the lungs and vapour delivery systems whereby the medicament is admixed to an inhalable vapour (often a water-containing vapour).

A vapour type pulmonary delivery system comprises a carrier liquid, often water or a water-glycol mixture (the glycol serving to stabilise the water droplets when in the vapour form) to which is admixed a desired medicament. The carrier liquid can be vaporised in various ways, such as by spraying it through a nozzle, but in many cases, it is simply heated to form a vapour comprising the carrier liquid and the desired medicament. The resultant vapour is then inhaled by a user to deliver the medicament. However, heating of the medicament can result in undesirable by-products being formed and thus, inhaled by the user. This may also reduce the accuracy of the dose of medicament inhaled.

An example of a vapour type pulmonary delivery system is an e-cigarette that vapes nicotine for inhalation by the user. A nicotine solution ("e-liquid") is provided in a reservoir (often in the form of a detachable cartridge) and passes along a wick to a heating element where it is vaporised and can be inhaled by the user. Generally, a length of resistance wire connected to a power source, such as a battery, is coiled around the wick. When activated the wire heats up, turning the e-liquid to vapour which is then inhaled by the user. Such a device has clear advantages over smoking conventional cigarettes since far fewer, and safer, ingredients are inhaled by the user than when smoking an ordinary tobacco cigarette. However, regular users have noted that the e-cigarettes do not "hit the spot" in the manner of a conventional tobacco cigarette. This is due to the wet vapour quickly condensing in the mouth of the user, resulting in the majority of nicotine absorption being through the mucous membranes of the nose, throat and airway leading to the lungs. In contrast, with conventional cigarette smoking, nicotine passes straight into the lungs giving a rapid absorption into the blood stream and a corresponding "quick hit".

An alternative type of e-cigarette is an atomizer inhaler. This type of device provides a better, more rapid absorption of nicotine due to the use of a cold, pressurized vapour in place of a heated vapour, thereby avoiding significant condensation of the vapour in the mucosa of the nose and throat. However, the cold and dry sensation provided by this type of device results in the overall experience differing greatly to smoking conventional cigarettes resulting in such devices being less popular than the vaping type e-cigarettes, resulting in none or poor compliance.

It is an aim of the present invention to provide an improved pulmonary delivery device that overcomes, or at least alleviates, the abovementioned drawbacks.

### SUMMARY OF THE INVENTION.

Accordingly, the present invention provides a pulmonary delivery device comprising: a first chamber adapted to thermally vaporise a quantity of a first liquid to form a relatively warm, wet first vapour and a second chamber adapted to atomize a quantity of a second liquid without heating of the second liquid to form a mist of a relatively cold, second vapour, and an outlet via which, in use, a user can inhale a mixture of the first and second vapours.

Preferably, the first chamber is provided with, or connected to, a heat source for vaporisation of the first liquid thereby creating a first "warm" vapour. In contrast, the second chamber includes an atomizer for delivery of a second "cold" vapour. At least one of the first and/or second liquids preferably contains an active molecule or medicament, such as nicotine. The second liquid may be the same or different to the first liquid. Preferably, the active molecule or medicament is included in the second liquid forming a "cold" vapour". In this manner, rapid absorption of an active molecule contained in the second vapour is achieved while providing the user with a desirable heat sensation on inhalation provided by the vapour of the "warm" first liquid.

Preferably, the first liquid comprises a carrier liquid (i.e. a liquid capable of forming a stable vapour), which may be an inert (non-medicated carrier liquid), such as water, or a water-glycol mixture. The second liquid, in an embodiment of the invention can comprise the desired active molecule or medicament. This has the added benefit that the active molecule or medicament is not in direct contact with a heat source reducing the potential for thermal degradation which may produce harmful by-products and reduce the efficacy of the medicament.

It is to be appreciated that is possible to control the composition of the mixture by controlling the quantity of vapour released from one or each of the chambers. Suitably, the delivery apparatus comprises a controller adapted to control, in use, the composition of the first and/or second vapours in the mixture, that is to say, by controlling the relative amounts of the first and second vapours in the mixture, or the ratio between the two. The controller could be adapted to switch one or the other of the vaporisers on or off, thus providing the option of delivering one or the other of the liquids in vapour form.

Thus, if the device is configured to deliver only the first liquid in vapour form to the user, the user receives a zero dose of the medicament. On the other hand, if the ratio of the first and second liquids is adjusted to a non-unity value, an amount of the second liquid can be added to the mixture inhaled by the user, thus delivering an accurate and defined dose of the medicament.

In another embodiment, the first and second liquids comprise different medicaments, and so the device can be adapted to vary the relative concentrations of the two, which may be particularly useful where, say, a course of drugs includes an initial dose of a first medicament, which transitions to a dose of a second medicament over a period of time.

Suitably, by enabling the device to control the ratio of one liquid to the other in the mixture, it is possible to vary the composition for the mixture in infinitesimal increments from 100% of the first liquid to 100% of the second liquid. Of course, if more than two vaporisers are provided in the device, then the ratios could be adjusted accordingly.

The first chamber preferably comprises a vaporiser in the form of an electric heater, for example a battery-powered resistive heating wire or coil. The current delivered to the resistive heating wire or coil can be used to control the temperature of the wire or coil, and thus regulate and/or control the heating and vaporisation of the liquids. In an embodiment of the invention, the heater comprises a hydrophilic or super-hydrophilic foil, which coats with a film of the liquid to be vaporised. A current can be passed through the coil to heat it, thereby vaporising the liquid. Alternatively, a ceramic heater may be used as the heat source. The use of a ceramic heater may be preferred as it reduces the potential for metals to be transferred/inhaled into the user's lung i.e. metal elements exposed to the high temperature may result in harmful metal residue being delivered to the lungs.

A feedback circuit is suitably provided to thermostatically regulate the temperature, or temperature profile of the heater. For example, a circuit may be provided to monitor the resistance of the wire or coil (the resistance being dependent on the wire or coil's temperature) and to adjust the current in the wire or coil such that the resistance, and hence the temperature, is controlled.

The vaporiser suitable for use in the first chamber of a pulmonary delivery device according to the present invention may comprise an electric heater adapted to vaporise a quantity of vaporisable liquid in contact therewith, the vaporiser further comprising a circuit configured to apply a time-dependent heating and/or cooling profile by temporally controlling an electric current in the heater in response to a measured temperature thereof.

Such a configuration, that is to say, a time-dependent heating and/or cooling profile, suitably controls the vaporisation of the liquid or liquids more precisely and reproducibly, and/or improves the longevity of the heater, which is suitably a heating wire, foil, coil or ceramic tube.

Other heating devices could equally be used, such as thermionic emitters, Peltier devices, infrared emitters and so forth, and the invention is not restricted to resistive heater wires, foils or coils.

The second liquid is vaporised by atomising or forcing a liquid through a nozzle or aperture to form a stable cold vapour or mist. This not only provides the user with rapid absorption of the active molecule but avoids potential degradation of the active ingredient which may expose the user to potential harmful by-products.

Any suitable atomiser may be incorporated into the device for forming the mist of the second vapour, such as an aerosol dispensing system, ultrasonic vibrators, compressors and electrical vibrating mesh technology. Preferably, the particles produced in the mist have an average diameter of less than 10 µm, more preferably being smaller than 5 µm to encourage deposition deeper into the lower airways.

By having the active molecule or medicament in the cold chamber, the dosage of the active can be controlled more accurately with reproducible dosage i.e. by not exposing the active molecule or medicament to high temperature and designing the atomization to be more accurate & reproducible.

In an embodiment of the present invention, the first and second chambers may comprise a dual-ended aerosol container, preferably encased in a housing with an outlet, wherein the first liquid is released from a first chamber forming part of the aerosol container and the second liquid is released from a second chamber forming part of the container. Preferably, the intended top end of the aerosol container releases the second cold vapour whereas the intended bottom end sprays the second liquid on to a heat source provided in the housing to warm the vapour which is then directed up towards the cold vapour and outlet.

It is to be appreciated that the delivery of the medicament to the lungs of the user may be controlled and/or varied by selection of the particle droplet size and velocity of the second vapour.

The controller for controlling the ratio of the first and second liquids in the mixture may be suitably programmable to change the ratio on a dose-by-dose basis. This may be accomplished using a microprocessor. In an embodiment of the invention, the controller comprises a computer, such as a System On Chip (SOC) device, which executes an application that controls the ratio. The computer is suitably adapted to interface with an external programming device. Suitably, the external programming device comprises a computer, which communicates with the device via a physical comms port (e.g. a USB port) or via a virtual comms port (e.g. a Wi-Fi, Bluetooth^{RTM} or WAN port).

In certain embodiments of the invention, the device can be pre-configured with a number of user-selectable programs. For example, the device may comprise a selector switch enabling the user to select between, say: 1) 100% of the first liquid and 0% of the second liquid; 2) 0% of the first liquid and 100% of the second liquid; and 3) a selected ratio of the two liquids, for example a 50%50% mix, a 90%-10% mix, and so on.

In another embodiment of the invention, the first vaporiser is always on, whereas the operation of the second vaporiser is user-initiated, for example, using a push button on the device.

Suitably, an interface is provided that enables a user, physician or health professional to configure the device to operate in various modes. The interface suitably comprises an application executed in an external computer, which is adapted to communicate with the device and thus to control and/or program it.

In an embodiment of the invention, for example, a nicotine weaning device, the pulmonary delivery system resembles a cigarette, a pipe or a cigar. In such a situation, the first liquid can comprise an inert mixture of water and glycol and the second liquid can contain a mixture of a propellant and the desired medicament, in this case, liquid nicotine. The device can thus be programmed to deliver a certain dose of medicament (nicotine) in each "puff" of the device, or over a given period, such as a day.

In the former case, the ratio of the first and second liquids is configured such that the inert liquid is always dispensed, whereas the dose of the second liquid is controlled to deliver only the pre-determined dose in each "puff". Thus, no matter how long, or how hard, the user draws on the device, only the predetermined amount of medicament (nicotine in this example) is delivered each time. This configuration usefully addresses the extrinsic dosage variations that arise from variations in users' physiology.

In the latter case, the dose can be configured to deliver a dose over a period of time, such as a day. In this case, the ratio of the first and second liquids can be dynamically configured such that for a given number of "puffs" per unit time, the user will be administered the predetermined dose.

In an embodiment of the invention, the timing of the "dose" can be configured. In one example, the device is configured to permit unlimited use of a first one of the liquids, but a metered and/or dosed consumption of the second liquid. The metering and/or dosing is suitably time-dependent, for example, allowing dosing two, three (or any desired number of) times per day, at specific times. The dose can also be configured to vary through the course of a day, for example, with higher doses in the morning and/or evening, say.

However, the device is suitably configured to monitor the number of puffs on the device, and to adjust the ratio of the first and second liquids to accord. Thus, the dose-per-puff of the medicament can be increased if the user is under-using the device, or reduced if the user is overusing the device, thus ensuring that the predetermined dose is not exceeded in the time interval. Such a configuration can usefully be used to ensure compliance with a prescribed dosage regimen, thereby negating the effects of poor user discipline. If the user under-uses the device, then the dose-per-puff is increased to compensate. Conversely, if the user over-uses the device, the dose-per-puff can be reduced to compensate. If the user over-uses the device excessively, and reaches the maximum dose level, the medicated liquid can be switched off, thereby allowing the user to continue to use the device, albeit in an inert mode, with no further delivery of medicament.

The device suitably logs and/or monitors the user's habits, and uses this information to re-configure the dosage in future time intervals. As such, an estimate of the number of puffs per day, say, can be adjusted up or down automatically, based on historic usage data. This usage data can also be fed back to health professionals, if desired, via the computer interface for reporting and/or monitoring purposes.

The vaporiser of the first chamber suitably comprises a reservoir for retaining, in use, a quantity of the respective liquid and a conveyor adapted to convey, in use, the liquid from the reservoir to a heater. In an embodiment of the invention, the reservoir comprises a vial and the conveyor comprises a wick extending between the interior of the vial and the heater. Suitably, a resistive heating wire, such as that described herein, can be wrapped or coiled around the wick to vaporise the liquid. The conveyor may comprise a capillary tube extending between the vial and the heater.

The vaporiser of the second chamber suitably comprises a reservoir for retaining, in use, a quantity of the respective liquid and a conveyor adapted to convey, in use, the liquid from the reservoir to an outlet. The vaporiser for the second liquid in the second chamber preferably comprises a reservoir containing a liquid under pressure that can be forced through an aperture to form a stable vapour. In an embodiment, this could comprise a pressure vessel containing the liquid and a propellant, and the conveyor comprises a flow control valve associated with the outlet of the pressure vessel. By opening the flow control valve for a predetermined interval, a predetermined quantity of the liquid can be dispensed.

Alternatively, the conveyor may comprise a piston of a syringe and the reservoir comprises a cylinder of a syringe. If the syringe comprises a mechanically-actuated syringe, such as a motorised syringe, the volume of liquid dispensed can be controlled by controlling the movement of the piston within the cylinder.

A further embodiment of the invention has a second chamber comprising a spring-loaded syringe-driver comprising a syringe cylinder containing a quantity of the liquid, a spring-biased piston adapted to force the liquid out of the cylinder via an outlet aperture and a flow control valve for selectively opening and closing the outlet aperture. Thus, the spring-loaded syringe driver is able to retain a quantity of the liquid, but also to deliver it in controlled doses by opening and closing the flow control valve.

The delivery of the liquid, however, will depend, to an extent on the position of the piston within the cylinder as the spring force is dictated by Hooke's law meaning that as the reservoir empties, the pressure applied to the liquid by the spring-loaded piston will decrease. This decrease in pressure can be compensated for by increasing the "open" time of the flow control valve.

In all of the cases above, the pressure within the reservoir (either by the pressurised propellant or by the spring force) can be configured to remain above the threshold value whereby the dispensation of the liquid is mass-limited. As such, the dose per actuation can be accurately controlled. In other words, if the quantity of liquid dispensed per actuation is mass-limited, the only variable is the triggering of the aperture/valve, such that the dose (quantity of liquid, having a fixed composition, dispensed) is directly proportional to the number of actuations of the valve/aperture.

The reservoir and/or conveyor is suitably configured such that the size of the reservoir, the pressure within the reservoir and the size of the reservoir outlet at its narrowest point are arranged such that the peak volumetric flow rate of the liquid for each dose with the valve fully open has a variation of less than 10%.

In another embodiment of the invention, the spring-loaded syringe driver comprises a superelastic spring, such as a nickel-titanium spring, that exhibits superelasticity over the range of the piston. Superelasticity is the phenomenon whereby a material exhibits a non-linear force-extension characteristic, and in some cases, having a constant force for a range of extensions, which overcomes, or at least partially addresses, the problem of force-fade with displacement of the piston.

Alternative atomization techniques may be incorporated into the device for forming the mist of the second vapour, such as an aerosol dispensing system, ultrasonic vibrators, compressors and electrical vibrating mesh technology.

The first and second liquids suitably comprise a solvent and a stabiliser. The stabiliser is suitably adapted to stabilise droplets of the solvent in air. The carrier liquid can comprise any one of more of the group comprising:
solubilizers, solvents and mixtures thereof such as water, alcohols such as glycerol, propylene glycol, polyethylene glycol, vegetable oils, mineral oils, lipids, cyclodextrins etc. Surface active agents such as anionic agents with carboxylate ions, sulphate groups and sulphonate groups; cationic surfactants, nonionic surfactants such as polyol ethers, polyoxyethylene esters and ethers, poloxamers; amphoteric surfactants, natural emulsifiers, sucrose esters and alkylpolyglucosides;
antioxidants such as ascorbic acid and its salts and derivatives tocopherols (vitamin E), thiol derivatives such as cysteine and acetyl cysteine, butylated hydroxyanisole (BHA), butylated hydroxytoluene (BHT), sodium hydrogen sulfite, sodium metabisulfite, sodium thiosulfate;
absorption enhancers such as alcohols, azone;
chelating agents, such as EDTA and galates;
minerals, such as fluorides;
propellants, such hydrofluroalkanes (HFA) Chlorofluorocarbons (CFCs), carbon dioxide etc.; sweeteners such as artificial sweeteners e g saccharin and its sodium and calcium salts, aspartame, acesulfame and its potassium salt, thaumatin and glycyrrhizin, polyhydric alcohols such as sorbitol, xylitol, mannitol and glycerol, glucose, fructose, galactose, sucrose, lactose, maltose and mixtures thereof;
flavourings and aroma such as essential oils of dried flowers, buds, leaves, stems, fruit, seeds, peel, bark, or root e g oil of peppermint, spearmint, eucalyptus, wintergreen, clove, cardamom, cinnamon, bitter almond, coriander, caraway, ginger, juniper, orange, bitter orange, lemon, grapefruit, bergamot, thyme, fennel rosemary etc., natural flavors and aroma agents of essential oils or concentrates of flavor components with natural origin from e g fruits, berries, nuts, spices, mints, tobacco, cocoa, coffee, tea, vanilla, liquorice, caramel, toffee, honey, wine, liquors and brews, synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews and mixtures thereof; and
pH regulators and buffering agents such as sodium, potassium or calcium hydroxide; bicarbonates, citrates and phosphates, etc.

One or both of the first or second liquids suitably comprise an active molecule or medicament. The active molecule, excipient or medicament may comprise any one or more of the pharmacologically active compounds from the group comprising:
H2-receptor antagonists such as cimetidine; and ranitidine;
Prostaglandin analogues such as misoprosol;
Proton pump inhibitors such as lansoprazole; omeprazole; and pantaprazole;
Agents to treat food allergies such as sodium cromoglicate;
Cardiac glycosides such as digoxin;
Diuretics such as amiloride; bendroflumethizide; indapamide; furosemide; hydrochlorothiazide; and xipamide;
Drugs for arrythmias such as procainamide; lidocaine; propranolol; atenolol; bisoprolol; carvedilol; pindolol; and nebivolol;
Antihypertensives and agents for treatment of angina such as clizapril; lisinopril; ramipril; trandolapril; amlodepine losartan; glyceryl trinitrate, isosorbide mononitrate; amlodipine; diltiazem; felodipine; isradipine; lacidipine etc.;
Lipid regulating drugs such as statins;
Drugs acting on the respiratory system such as salbutamol; terbutaline; bambuterol;
Antihistamines such as cinnarazine; promethazine; perphenazine and prochlorprazine;
Hypnotics such as zolpidem; zopiclone; clomethiazole;
Anxiolytics such as benzodizapines; buspirone;
Antipsychotic agents such as benperidol;fluphenazine; pimozide and amisulpride;
Antidepressant drugs such as tricyclics; mianserin; MAOIs; SRIs; reboxetine etc.;
Central nervous system (CNS) stimulants such as methylphenidate;
Drugs used in the treatment of nausea such as antihistamines; domperidone; metoclopramide; 5HT3 antagonists; hyoscine, and betahistine;
Opiod analgesics such as morphine; buprenorphine and fentanyl;
Anti-migraine drugs such as 5HT1 agonist and ergot alkaloids;
Drugs used in treatment of Parkinsonism such as apomorphine; bromocriptine; lisuride; haloperidol and ergot alkaloids;
Drugs used in substance dependence such as nicotine and buprenorphine;
Drugs used in dementia such as rivastigmine; dihydroergotamine; dihydroergocristine and dihydroergocryptine;
Antibiotics; antifungals; antivirals and antimalarials;
Drugs used in treatment of diabetes;
Glucocorticoid therapy using steroids such as betamathasone and dexamethasone;
Male and/or female sex hormones such as estradiol; norethisterone; progesterone; testosterone and esters;
Pituitary hormones such as vasopressin and desmopresin;
Drugs affecting bone metabolism such as calcitonin and bisphosphonates;
Endocrine drugs such as bromocriptine and cabergoline;
Contraceptives such as oestrogens; progestrogens and combinations thereof;
Drugs used in urinary frequency and enuresis such as oxybutinin and desmopressin;
Drugs used in erectile dysfunction such as apomorphine and sildenafil;
Drugs used in malignant disease and immunosuppresion such as buslfan; antimetabolites; alkaloids; corticosteriods; hormones and interferons;
Non-steroidal anti-inflammatory drugs such as diclofenac; piroxicam and refoxicab;
Drugs used in treatment of gout such as colchicines;
Drugs used in neuromuscular disorders such as neostigmine and pyridostigm ine;
Muscle relaxants such as diazepam; tizanidine;
Vaccines delivered by subcutaneous route;
Agents for the treatment of nicotine withdrawal symptoms such as nicotine; and
Cannabinoids.

The at least one active compound may be a nutraceutically active compound. A "nutraceutically active compound" is a compound, derived from a natural origin (animal or vegetable) that has a beneficial and/or therapeutic effect on the human or animal body in the treatment of a condition. Such compounds may be regarded as nutrients.

Suitable nutraceutically active compounds may be natural products extracted from animals or vegetables. Examples of suitable nutraceutically active compounds include:
Carotenoids such as lycopene, lutein, astaxanthin and □-carotene; glucosamine or Nacylglucosamine; ubiquinone;
Vitamins such as vitamins A, C, D and E; Rosmarinic acid; Honokiol; Magnolol; Chlorogenic acid; Oleuropein; Methylsulphonylmethane ("MSM"); Collagen and Chondroitin; Boswellin and boswellic acid; Escin and esculin; Tumeric extracts such as curcuminoids and etrahydrocurcuminoids; Gingerol and gingerone; Triterpenes such as ursolic acid and oleanolic acid; Diterpenes such as asiaticoside, sericoside and ruscogenins; Hydroxycitric acid ("HCA") and niacinamide hydroxycitrate;Trigonellin; and Corosolic acid; Saw palmetto; and St John's Wort.

The device suitably comprises a battery, such as a rechargeable battery, for powering the heater of the first chamber and/or control circuitry.

The heater is suitably switched on or off using a switch. The switch is suitably an automatic switch that is triggered by the user inhaling on the device. The switch may therefore comprise a pressure-activated switch associated with the outlet of the device, whereby when a user draws on the device, the switch is turned on thereby automatically switching on the heater, and whereby the heater is switched off again when the user ceases drawing on the device. Suitably, the device additionally comprises a second pressure-sensitive switch for monitoring the pressure of the ambient air.

The second chamber containing the second liquid may also be breath-activated.

Another aspect of the invention provides a pulmonary delivery apparatus comprising: a first reservoir for retaining, in use, a quantity of a first liquid; a first conveyor adapted to convey, in use, the first liquid from the first reservoir to a heater adapted, in use, to heat a quantity of the first liquid to form a first relatively warm vapour, a second reservoir for retaining, in use, a quantity of a second liquid under pressure and a pressure release valve adapted, in use, to propel a quantity of the second liquid to form a second relatively cold vapour, and an outlet through which, in use, a user can extract, by inhalation, a mixture of the first and second vapours from the apparatus.

### BRIEF DESCRIPTION OF THE DRAWINGS.

Embodiments of the invention shall now be described, by way of example only, with reference to the accompanying drawings in which:
Figure 1 is a perspective view of a pulmonary delivery device in accordance with the invention;
Figure 2 is an exploded view of the pulmonary delivery device of Figure 1;
Figure 3A is a plan view of the pulmonary delivery device of Figure 1;
Figure 3B is a close up view of part of Figure 3A;
Figure 4 is a plan view of another pulmonary delivery device in accordance with the invention; and
Figure 5 is a schematic view of a pulmonary delivery device and user interface in accordance with the invention.

### DETAILED DESCRIPTION.

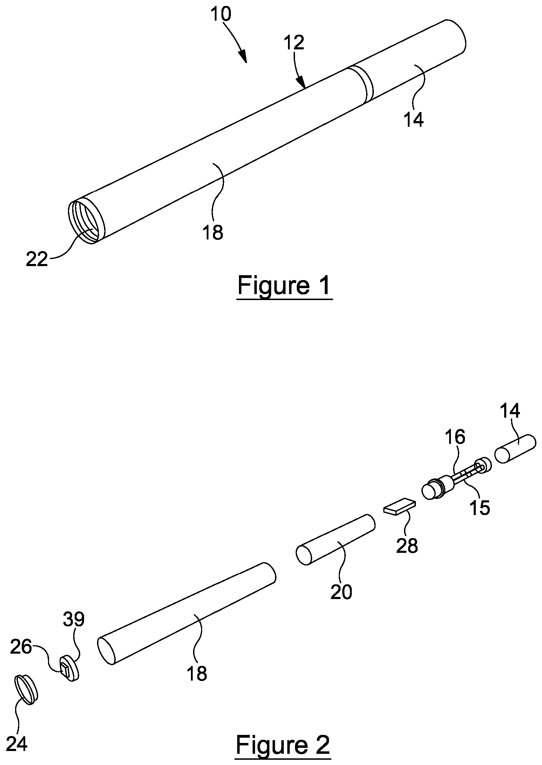

Referring to Figures 1, 2 and 3A to 3B of the accompanying drawings, a pulmonary delivery device 10 comprises a generally cylindrical main body portion 12 adapted to resemble a cigarette. The main body portion 12 comprises a tubular filter chamber 14 encasing dual vaporiser chambers 15, 16 and a tubular battery chamber 18 encasing a rechargeable battery 20. The tip 22 of the main body 12 is closed off by a translucent end cap 24, behind which sits an LED indicator light 26 that illuminates when the device 10 is in use. A control circuit 28 is contained within the body 12, which comprises a programmable circuit for controlling the operation of the device 10, in use.

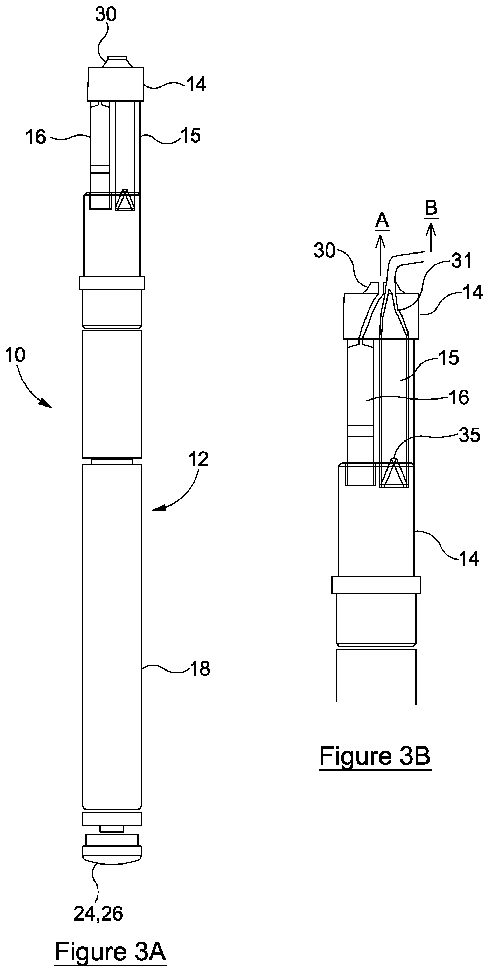

Turning to Figures 2 and 3, the device 10 comprises a first pressure sensor (not visible) located within the filter chamber 14, which has an outlet aperture 30 therein through which, in use, vapour generated by the device 10 can be inhaled by a user. When the user draws on the filter chamber 14, the pressure sensor (not visible) activates the first and/or second vaporisers 15, 16 to form a mixed vapour comprising the first and/or second liquids, to be inhaled.

The dual vaporiser chambers 15, 16 comprise a pair of separate reservoirs containing first and second liquids respectively. A first reservoir 15 contains a first liquid and comprises a capillary wick 31, which absorbs the liquid, and whose end touches a heater element in the form of a pyramid-shaped, super-hydrophilic foil 35, which is wetted by the first liquid, in use (see, in particular, Figure 3B). The heater element 35 could alternatively comprise a resistive heating coil, which is wrapped around the wick 31. In any event, the heater element 35 is connected to the battery 20 under the control of the control circuit 28.

The second reservoir 16 contains a second liquid that is held under pressure within the reservoir and includes a pressure release valve or flow control valve (not shown). When the user draws on the filter chamber, the pressure sensor activates the valve to propel the second liquid out of the second reservoir as a fine mist or vapour. The absence of any heating element results in a cold vapour being released from the second reservoir.

Thus, when the heater 35 is switched on, the first chamber acts as a "warm vapour chamber" with the first liquid being evaporated and forming a warm vapour B within the interior of the filter chamber 14. Simultaneously, cold vapour A is released into the interior of the chamber 14 from the second reservoir (the "cold vapour chamber") thus allowing the warm and cold vapours B, A to mix in the hollow space of the filter chamber, before being inhaled by the user, via the outlet aperture 30 of the device.

In a preferred embodiment of the present invention, the first liquid comprises a mixture of glycerol and water and the second liquid comprises nicotine and a suitable propellant. Preferably, the particles forming the mist of the second liquid are less than 10 µm in diameter, more preferably less than 5 µm. In this manner, nicotine (or other active molecule provided in the second liquid) is delivered deep into the lungs to allow for its quick absorption into the bloodstream via the lungs. However, the simultaneous delivery of a warm, wet vapour in the form of the vaporised first liquid provides the user with a sensation that more closely resembles that experienced during the smoking of a conventional tobacco cigarette. The active molecule is not in direct contact with the heater element, reducing the potential for its thermal degradation which may have resulted in the user inhaling harmful by-products. In contrast, only glycerol and water are in contact with the heater element which do not result in the production of harmful by-products upon their thermal degradation.

The device may also be provided with a suitable control circuit 28 that may control the delivery of the first and/or second vapours from their respective chambers. The ability to deliver nicotine from a pressurized chamber without heating allows for more accurate nicotine dosing using the device of the present invention than the delivery of nicotine using the heated vapour method. It is to be appreciated that the delivery of the wet warm vapour and the cold vapour may be controlled and the content of the mixed vapour may be adjusted as required.

For example, the control of the delivery of heated vapour may be achieved using a resistance sensor operatively connected to the heater element 35 which measures the heater's resistance, and infers from that, the heater temperature. The control circuit 28 additionally comprises a current limiting circuit for limiting the current to the heater 35 and is programmed to heat it according to a predetermined, time-dependent heating/cooling profile.

When a user draws on the filter chamber 14, the pressure switch (not visible) triggers the control circuit 28 to heat the heater 35. The control circuit 28 thereby connects the battery 20 to the heater 35 in a controlled and reproducible manner. As such, the time, and time-at-temperature of the heater 35 is thus controlled, thereby regulating the vaporisation of the first liquid from the wick 31.

The control circuit 28 may also be operatively connected to a second pressure sensor 39 (Figure 2), which measures the ambient air pressure. The control circuit 28 is configured to switch on the heater 35 only when the first pressure switch is triggered, as described hereinabove.

A vapour is thereby formed adjacent the heater within a hollow interior space (a mixing chamber) located towards the tip of the filter chamber 14, i.e. the space between the vaporiser chamber 15 and the outlet aperture 30, when the device 10 is assembled.

The outlet aperture from the "cold vapour chamber" is sufficiently small as to control (mass-limit) the amount of the second liquid that can escape in each dispensation, and is selectively closed and/or opened by a control valve (not shown). The control valve is connected to the control circuit 28 enabling it to be controlled independently of the heater element. The control circuit 28 can thus be configured to open the valve a given number of times, per actuation, thereby incrementally controlling the dose of liquid dispensed (the dose per actuation being constant due to the size of the outlet aperture). Thus, the device is able to accurately control the ratio of the first and second liquids dispensed in each actuation, and hence the dose of a particular medicament or mixture of medicaments in the first and second liquids. The ratio may be adjusted by the control circuit 28, in accordance with a pre-programmed dosing regimen.

While the device of the present invention may accurately control the dose provided by each chamber, if the active molecule or medicament is only included in the "cold vapour" chamber then it is possible to only accurately control dosing provided by this chamber. This allows for simpler control of dosing than when the active molecule or medicament is dispensed in the warm vapour.

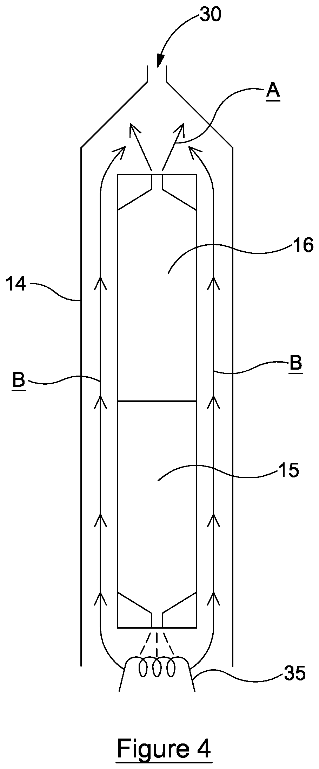

Figure 4 shows an alternative pulmonary delivery device in accordance with the invention. In Figure 4, the first and second chambers are not in a side-by-side arrangement but instead arranged along the longitudinal axis of the device, in an end-to-end arrangement, thereby providing a slim-line device. Features that are identical to those described in Figures 1 to 3 are given the same reference numerals, for the sake of simplicity. The chambers 15, 16 are provided in a hollow cylindrical pressure vessel comprising a dual ended aerosol container having an aerosol outlet at each end, the container being surrounded by housing 14 having outlet 30. The cold vapour chamber 16 is provided in the intended top end of the aerosol container near to the outlet 30 and the warm vapour chamber 15 extends from the base of the chamber 16 in the bottom end of the aerosol container. A heating element 35 is provided in line with the base of the aerosol container. The second liquid containing the active molecule is dispensed from the upper chamber (A) and the first liquid is dispensed from the lower chamber on to the heating element 35 (B). This warms up the second vapour which then passes up the passage (B) between the housing and container to the outlet 30, thereby enabling mixing of the hot and cold vapours (B, A) prior to their exit through the outlet and inhalation by the user.

In this embodiment, the control circuit may be programmed to activate vapourisation of the first liquid in the warm vapour chamber a few milliseconds before release of vapour from the cold vapour chamber thereby ensuring that warm vapour is released simultaneously with the cold vapour.

It is to be appreciated that alternative arrangements may be provided for the warm and cold vapour chambers in the device of the present invention. For example, the device may include a dual chamber having a hollow cylindrical pressure vessel comprising a central divider thus dividing the interior thereof into two separate reservoirs for first and second liquids. A pressurised propellant gas occupies the remaining space of one reservoir and a heater element and wick is provided in the other reservoir, with outlet apertures provided to enable the liquids to escape from their respective reservoirs under the actions of the pressurised propellant and heater.

In a preferred embodiment of the present invention, a ceramic heater is used for heating the first liquid in the first chamber. This reduces the potential for harmful metal residues from metallic heating elements to be inhaled by the user.

An alternative type of "cold vapour" chamber may comprise a spring-loaded syringe comprising a tubular body portion forming a reservoir for retaining the second liquid. A piston is slideably moveable within the body and is sealed thereto by an O-ring seal. A superelastic spring cooperates between the rear face of the piston and an end cap of the body to push the piston along the body and thus eject the liquid contained therein through an outlet aperture. An outlet flow control valve is also provided to open and close the outlet aperture. The superelastic spring is compressed within its superelastic range and, provided the superelastic spring is operated within this range, the pressure of the liquid remains constant, thereby accurately regulating the amount of liquid dispended during each actuation of the valve.

A device incorporating this type of "cold vapour" vaporiser would, of course, also include a "warm vapour" chamber with a heating element for releasing a warm vapour, and, optionally, a control circuit 28 to control the delivery of the liquids.

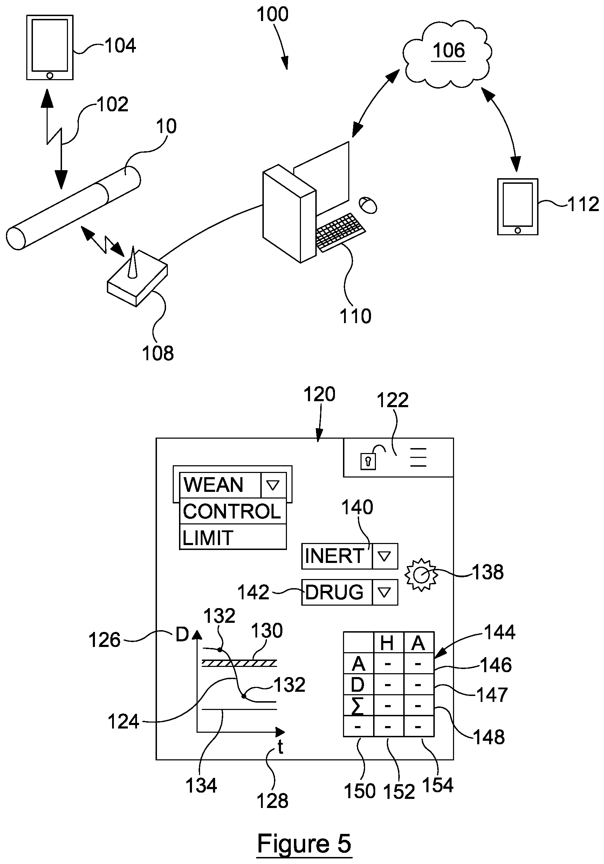

A dose control system 100 is shown in Figure 5 of the drawings. In Figure 5, a pulmonary delivery device 10 according to the invention is wirelessly connected 102 to a user's smartphone, tablet computer or PC 104 and to the internet 106, via a Wi-Fi access point 108, such as a broadband router. Internet connected computers 110, 112 (local or remote) can thus connect to the device 10 wirelessly, as can the user him or herself. The wireless connection is provided via a Wi-Fi and/or Bluetooth^{RTM} interface of the control circuit 28, thereby providing a graphical user interface (GUI) 120 on any of the devices 104, 110, 112 for interacting with the device 10.

The GUI 120 has a secure login-in system 122 to prevent unauthorised reconfiguration of the device 10 and allows a user to select between three main modes of operation, namely a "wean" mode 124 whereby the dose 126 of a given medicament can be reduced over time 128, as shown on a dose-time graph 130 of the GUI. The graph has dragable handles 132 that enable the shape of the curve to be adjusted to change the weaning profile 124, i.e. the severity, duration, delay etc. of the weaning process.

Another option from the drop-down menu is to select a control program 134, which ensures that a desired quantity of medicament is administered over a period of time. The dose-per-puff is thus controlled to ensure, on average, a relatively even administration of the medicament over the time period.

A third option is to set an upper limit 136, which may be useful in analgesic applications. This program prevents a maximum dose per unit time from being delivered, but allows for under-administration.

The GUI 120 comprises a configuration settings menu 138, which enables a user to configure the GUI in accordance with the liquids 140, 142 in the device. A history table 144 is also provided, which provides a summary of the number of administrations 146, the amount of medicament delivered 147 and a running total 148. These data are shown on a historic 150, actual 152 and a target 154 basis to facilitate monitoring of the drug delivery to the user.

The invention is not restricted to the details of the foregoing embodiments, which are merely exemplary of the invention. For example, the shape and configuration of the device can be changed, the materials of manufacture, the combinations of vaporiser technology used, the combinations of heaters used, the additional features, such as the on/off switch, control valves etc. can be varied without departing from the invention.

For example, the first "warm vapour" reservoir containing an inert liquid, such as a water-glycol mixture, which forms an inhalable vapour may be consumed without restriction by a user. The device has a pressure switch located within the filter tube 14, which detects when a user inhales on the device. The pressure switch is connected to the control circuit 28, and the control circuit is adapted to switch on a current, from the battery 20, to a resistive heating coil wrapped around an end of the wick, which evaporates the first liquid to form a vapour that can be drawn from the device, via the vaporiser outlet and the device's main outlet aperture 30.

The device may additionally comprise a push switch that is accessible from the exterior of the device, which a user can depress, in use, to actuate the valve of the "cold vapour" chamber. Thus, a user can use the device at will, and can choose when to administer a dose of a medicament or active ingredient, such as nicotine, which is contained in the second reservoir, by pressing on the button during inhalation.

The device of the present invention provides many potential advantages over earlier pulmonary delivery devices. The active ingredient, such as nicotine or a cannaniboid, is inhaled as small particles (< 10µm) resulting in it being delivered deep into the lungs of a user enabling its fast absorption into the bloodstream. The simultaneous delivery of a warm inert vapour enhances the flavour and sensation of the inhalation. The active ingredient is not subject to thermal degradation, leading to a reduction in any harmful by-products and increasing the accuracy and reproducibility of the dosage.

The invention may be described by reference to the following numbered paragraphs:-
1. A pulmonary delivery device comprising: a first chamber adapted to thermally vaporise a quantity of a first liquid to form a relatively warm first vapour and a second chamber adapted to atomize a quantity of a second liquid without heating of the second liquid to form a mist of a relatively cold second vapour, and an outlet via which, in use, a user can inhale a mixture of the first and second vapours.
2. The delivery device of paragraph 1 above wherein the first chamber is provided with, or connected to, a heat source for vaporisation of the first liquid.
3. The delivery device of paragraph 1 or 2 above wherein the second chamber is provided with an atomizer for delivery of the second vapour.
4. The delivery device of paragraphs 1, 2 or 3 above, wherein at least one of the first and/or second liquids contains an active molecule or medicament, preferably the active molecule or medicament is included in the second liquid forming the cold second vapour.
5. The delivery device of any one of paragraphs 1 to 4 above wherein the first liquid comprises a carrier liquid (i.e. a liquid capable of forming a stable vapour), preferably being an inert (non-medicated carrier liquid), such as water, or a water-glycol mixture.
6. The delivery device of any one of paragraphs 2 to 5 above wherein the heat source of the first chamber comprises an electric heater selected from the group consisting of a battery-powered resistive heating wire or coil, a hydrophilic or super-hydrophilic foil and a ceramic heater.
7. The delivery device of paragraph 6 above further comprising a circuit configured to apply a time-dependent heating and/or cooling profile by temporally controlling an electric current in the heater in response to a measured temperature thereof.
8. The delivery device of any one of paragraphs 2 to 5 above wherein the heat source of the first chamber is selected from the group consisting of thermionic emitters, Peltier devices and infrared emitters.
9. The delivery device of any one of paragraphs 1 to 8 above wherein the second liquid is vaporised by atomising or forcing a liquid through a nozzle or aperture to form the mist of the second vapour.
10. The delivery device of any one of paragraphs 1 to 9 above wherein the second chamber includes an atomiser selected from the group consisting of an aerosol dispensing system, ultrasonic vibrator, compressor and electrical vibrating mesh dispensing system.
11. The delivery device of paragraph 9 or 10 above wherein particles produced in the mist of the second vapour have an average diameter of less than 10 µm, more preferably less than 5 µm.
12. A delivery of paragraph 1 above wherein the first chamber has a vaporiser comprising a reservoir for retaining, in use, a quantity of the respective liquid and a conveyor adapted to convey, in use, the liquid from the reservoir to a heater.
13. A delivery device of paragraph 12 above wherein the reservoir comprises a vial and the conveyor comprises a wick extending between the interior of the vial and the heater and a resistive heating wire or coil is wrapped or coiled around the wick to vaporise the liquid.
14. A delivery device of paragraph 1 above wherein the second chamber has a vaporiser comprising a reservoir for retaining, in use, a quantity of the respective liquid and a conveyor adapted to convey, in use, the liquid from the reservoir to an outlet.
15. A delivery device of paragraph 14 above wherein the reservoir contains a liquid under pressure that is adapted to be forced through an aperture to form a stable vapour.
16. A delivery device of paragraph 15 above wherein the reservoir comprises a pressure vessel containing the liquid and a propellant, and the conveyor comprises a flow control valve associated with the outlet of the pressure vessel.
17. A delivery device of paragraph 14 above wherein the conveyor comprises a piston of a syringe and the reservoir comprises a cylinder of a syringe.
18. A delivery device of paragraph 17 above wherein the second chamber comprises a spring-loaded syringe-driver comprising a syringe cylinder containing a quantity of the liquid, a spring-biased piston adapted to force the liquid out of the cylinder via an outlet aperture and a flow control valve for selectively opening and closing the outlet aperture.
19. A delivery device of any one of paragraphs 1 to 11 above wherein the first and second chambers comprise a pressure vessel, each chamber having a release valve and containing the first or second liquid respectively and a propellant, the first chamber being adapted to deliver the vapourised first liquid released via the release valve to a heat source.
20. A delivery device of paragraph 19 above wherein the pressure vessel comprises a dual ended aerosol container having a chamber containing the second liquid at the intended top end of the aerosol container and a chamber containing the first liquid at the intended bottom end thereof, the heat source being provided in line with the release valve of the bottom chamber.
21. The delivery device of paragraphs 1 to 20 above further comprising a battery, such as a rechargeable battery, for powering a heater of the first chamber and/or any control circuitry.
22. The delivery device of paragraphs 1 to 21 above further comprising a controller adapted to control, in use, the composition of the first and/or second vapours in the mixture, that is to say, by controlling the relative amounts of the first and second vapours in the mixture, or the ratio between the two.
23. The delivery device of paragraph 22 above wherein the controller is adapted to switch one or the other of the vaporisers on or off, thus providing the option of delivering one or the other of the liquids in vapour form.
24. The delivery device of paragraph 22 or 23 above wherein the controller for controlling the ratio of the first and second liquids in the mixture is suitably programmable to change the ratio on a dose-by-dose basis.
25. The delivery device of paragraph 24 above wherein the device can be pre-configured with a number of user-selectable programs.
26. The delivery device of any of paragraphs 1 to 25 above wherein the first vaporiser is always on, whereas the operation of the second vaporiser is user-initiated, for example, using a push button on the device or being breath-activated.
27. The use of a device of any of paragraphs 1 to 26 above for the delivery of nicotine.
28. The use of a device of paragraph 27 above wherein the device resembles a cigarette, a pipe or a cigar.
29. The use of a device of paragraph 27 or 28 above wherein the first liquid comprises an inert mixture of water and glycol and the second liquid comprises a mixture of a propellant and liquid nicotine.
30. A pulmonary delivery apparatus comprising: a first reservoir for retaining, in use, a quantity of a first liquid; a first conveyor adapted to convey, in use, the first liquid from the first reservoir to a heater adapted, in use, to heat a quantity of the first liquid to form a first relatively warm vapour, a second reservoir for retaining, in use, a quantity of a second liquid under pressure and a pressure release valve adapted, in use, to propel a quantity of the second liquid to form a second relatively cold vapour, and an outlet through which, in use, a user can extract, by inhalation, a mixture of the first and second vapours from the apparatus.

## Claims

1. A pulmonary delivery device comprising: a first chamber adapted to thermally vaporise a quantity of a first liquid to form a relatively warm first vapour and a second chamber adapted to atomize a quantity of a second liquid without heating of the second liquid to form a mist of a relatively cold second vapour, and an outlet via which, in use, a user can inhale a mixture of the first and second vapours.

2. The delivery device of claim 1, wherein at least one of the first and/or second liquids contains an active molecule or medicament, preferably the active molecule or medicament is included in the second liquid forming the cold second vapour.

3. The delivery device of claim 1 or 2, wherein the first liquid comprises a carrier liquid (i.e. a liquid capable of forming a stable vapour), preferably being an inert (non-medicated carrier liquid), such as water, or a water-glycol mixture.

4. The delivery device of claim 1, claim 2 or claim 3, wherein the heat source of the first chamber comprises an electric heater selected from the group consisting of a battery-powered resistive heating wire or coil, a hydrophilic or super-hydrophilic foil and a ceramic heater.

5. The delivery device of claim 4 further comprising a circuit configured to apply a time-dependent heating and/or cooling profile by temporally controlling an electric current in the heater in response to a measured temperature thereof.

6. The delivery device of any one of claims 1 to 3 wherein the heat source of the first chamber is selected from the group consisting of thermionic emitters, Peltier devices and infrared emitters.

7. The delivery device of any one of the preceding claims wherein the second liquid is vaporised by atomising or forcing a liquid through a nozzle or aperture to form the mist of the second vapour.

8. The delivery device of any one of the preceding claims wherein the second chamber includes an atomiser selected from the group consisting of an aerosol dispensing system, ultrasonic vibrator, compressor and electrical vibrating mesh dispensing system.

9. A delivery device as claimed in claim 1 wherein the first chamber has a vaporiser comprising a reservoir for retaining, in use, a quantity of the respective liquid and a conveyor adapted to convey, in use, the liquid from the reservoir to a heater, preferably wherein the reservoir comprises a vial and the conveyor comprises a wick extending between the interior of the vial and the heater and a resistive heating wire or coil is wrapped or coiled around the wick to vaporise the liquid.

10. A delivery device as claimed in claim 1 wherein the second chamber has a vaporiser comprising a reservoir for retaining, in use, a quantity of the respective liquid and a conveyor adapted to convey, in use, the liquid from the reservoir to an outlet, preferably wherein the reservoir contains a liquid under pressure that is adapted to be forced through an aperture to form a stable vapour.

11. A delivery device as claimed in claim 1 wherein the first and second chambers comprise a pressure vessel, each chamber having a release valve and containing the first or second liquid respectively and a propellant, the first chamber being adapted to deliver the vapourised first liquid released via the release valve to a heat source, preferably wherein the pressure vessel comprises a dual ended aerosol container having a chamber containing the second liquid at the intended top end of the aerosol container and a chamber containing the first liquid at the intended bottom end thereof, the heat source being provided in line with the release valve of the bottom chamber.

12. The delivery device as claimed in any one of the preceding claims further comprising a controller adapted to control, in use, the composition of the first and/or second vapours in the mixture, that is to say, by controlling the relative amounts of the first and second vapours in the mixture, or the ratio between the two.

13. The delivery device as claimed in any one of the preceding claims wherein the first vaporiser is always on, whereas the operation of the second vaporiser is user-initiated, for example, using a push button on the device or being breath-activated.

14. The use of a device as claimed in any one of the preceding claims for the delivery of nicotine.

15. The use of a device as claimed in claim 14 wherein the first liquid comprises an inert mixture of water and glycol and the second liquid comprises a mixture of a propellant and liquid nicotine.
